Europäisches Patentamt

European Patent Office

Office européen des brevets

(11) Publication number: **0 209 331**
**A2**

## EUROPEAN PATENT APPLICATION

(21) Application number: 86305339.3

(22) Date of filing: **11.07.86**

(51) Int. Cl.4: **C07K 7/40** , **A61K 37/36** , **C07K 1/14**

(30) Priority: **17.07.85 US 756406**

(43) Date of publication of application:
**21.01.87 Bulletin 87/04**

(84) Designated Contracting States:
**BE DE FR GB IT LU NL**

(71) Applicant: **PFIZER INC.**
**235 East 42nd Street**
**New York, N.Y. 10017(US)**

(72) Inventor: **Palmer, John Ligon**
**150, West Evergreen Avenue Apt. D3**
**Philadelphia Pennsylvania 19118(US)**

(74) Representative: **Graham, Philip Colin**
**Christison et al**
**Pfizer Limited Ramsgate Road**
**Sandwich, Kent CT13 9NJ(GB)**

(54) **Purified bovine somatomedin C.**

(57) Purification of bovine somatomedin C (BSM-C) by sequential reverse phase chromatography in various systems followed by Sephadex chromatography, high performance liquid chromatography - (HPLC), fast protein liquid chromatography and further HPLC affords approximately a seven million-fold purification.

EP 0 209 331 A2

# PURIFIED BOVINE SOMATOMEDIN C

This invention relates to the peptide growth promoter bovine somatomedin C (BSM-C). More particularly it relates to a process for purifying BSM-C and to the purified BSM-C thus obtained.

The somatomedins are a group of related peptide growth promoters with mitogenic properties and insulin like effects which mediate the action of growth hormone on its target tissues. They cause increased protein and DNA synthesis in metabolically active tissues and increased incorporation of sulfate into chondroitin sulfate, especially in cartilage and skeletal tissues.

Salmon et al., J. Lab. Clin. Med. 49, 825-836 - (1957) found that growth hormone (GH) was incapable of increasing the uptake of $^{35}SO_4$ in primary cartilage cultures. Similarly, serum from hypophysectomized rats (in which the source of growth hormone is excised) was ineffective in stimulating sulfate uptake above basal levels. A marked stimulation was observed, however, when normal serum or serum from growth hormone treated hypophysectomized rats was added. This observation of growth hormone stimulated production of a sulfation factor led to a search for other mediators of the growth stimulating activity of growth hormone. These are grouped together as the somatomedins (SMs).

Several somatomedins, all closely related chemically and biologically and having molecular weights of about 7,500 daltons, have been isolated from serum from different sources. SM-A, first isolated by Fryklund et al., Biochem. & Biophys. Res. Commun. 61, 957 (1954) was shown to be a neutral peptide of 7,500 daltons and appeared to lack disulfide bonds. SM-B, isolated by Fryklund et al. FEBS Lett. 87, 55-60 (1978) is no longer considered to be a SM. SM-C, a basic peptide with molecular weight of 7,500 daltons, was isolated by Svoboda et al., J. Biol. Chem., 19, 790-797 (1980). Rinderknecht etal., Proc. Nat. Acad. Sci. 73, 2365, 4379 (1976) described the isolation of two polypeptides with "non-suppressible insulin-like activity": NSILA I and NSILA II. These activities were not removed by anti-insulin antibodies, yet they exhibited insulin-like properties in stimulation of glucose oxidation in rat epididymal fat pads. These growth factors have since been renamed IGF I and IGF II for insulin-like growth factors I and II. The total sequence of human IGF-I and its similarity to human proinsulin was reported by Rinderknecht et al., FEBS Lett. 89, 283-286 (1978). Klapper et al., Endocrinology 112, 2215-2217 (1983) elucidated the total sequence of human SM-C and found it to be identical to the sequence reported earlier for IGF-I.

A further growth promoting substance referred to as multiplication stimulating activity (MSA) was isolated by Pierson et al. J. Cell. Physiol. 79, 319-330 (1972) as the active component of calf serum in the promotion of cell growth in culture. MSA may be isolated by procedures similar to that for somatomedin C and has a molecular weight of 4,000 to 5,000 daltons. It is highly active on chick and rat embryo fibroblasts and on human diploid cells stimulating DNA synthesis and cellular proliferation. Its effects on administration to the whole animal are as yet undetermined.

Van Wyk et al., in Growth Hormone and Other Biologically Active Peptides," Excerptica Medica, Amsterdam, ed. Pecile and Muller report the isolation and purification of SM-C from Cohn Fraction IV of human plasma.

Liberti, Biochem. Biophys. Res. Commun. 67, 1226-1233 (1975), reports on the partial purification, from bovine serum, of a factor which exhibited some of the chemical and physical properties of bovine somatomedin C (BSM-C). However, the identity of the isolated material, about 4% pure, was not established. Liberti notes the material isolated could be NSILA-S or MSA.

The site of biosynthesis of the somatomedins, including BSM-C, is not known with certainty. They may, in fact, be made by all tissues. Somatomedin C in circulating plasma is at a higher concentration than can be extracted from any organ tested.

The potential value of BSM-C as a growth promotant in the field of animal husbandry renders self-evident the need for an adequate supply thereof. This, in turn, renders axiomatic the need for a means of obtaining BSM-C in sufficient purity and amount to permit its use for the purpose of growth stimulation in animals particularly in the beef industry.

This invention provides a method for the isolation in a highly purified form, hence purification, of BSM-C from bovine serum and/or bovine plasma. The method comprises centrifuging beef blood to remove whole blood cells, ultrafiltration of the plasma through a hollow fiber filter, concentration of the filtrate followed by isoelectric precipitation of proteins from the concentrate and sequential C-18 reverse phase chromatography of the supernatant under varying conditions. Further purification was achieved by gel chromatography on Sephadex G-50 followed by high performance liquid chromatography (HPLC), fast protein liquid chromatography - (FPLC) and further HPLC.

## Plasma Collection and Ultrafiltration

Bovine blood (200 l.) obtained from a local slaughter house was made 10 mM in ethylenediaminetetraacetic acid disodium salt to prevent clotting and then centrifuged at 10,000 g for a half-hour to separate the whole blood cells. (If any clotting is observed, the blood is filtered through a cloth laundry bag prior to centrifuging).

The collected plasma was diluted 1:1 with water, made 5% in formic acid, then ultrafiltered on a Amicon DC30 using an H5P50-43 hollow fiber filter (50,000 molecular weight cut-off). 600 liters of filtrate were collected using 5% formic acid as the make-up. At this point addition of formic acid was halted and an additional 100 liters of filtrate were collected.

The DC30 was then washed with 0.1N NaOH and rinsed with water to remove all, remaining protein. The 50,000 MW filtrate was then concentrated to 21 liters using the DC 30 in conjunction with a H10P3-20 hollow fiber filter.

## Isoelectric Precipitation

The formic acid solution was adjusted to pH 5.0 with concentrated sodium hydroxide and centrifuged at 6,000 g for 60 minutes to remove the precipitate which formed. The supernatant was then brought to pH 7.1 with concentrated NaOH at which point further precipitation occurred. The precipitate was removed by centrifugation at 6,000 g for 30 minutes.

## C-18 Reverse Phase Chromatography-pH 7.16

A 5x90 cm C-18 column was prepared using Waters PrepPAK-500 C-18. The column was poured and washed with 8 liters acetonitrile followed by 8 liters 10mM sodium phosphate (pH 7.16). Three column runs were required to process the pH 7.1 supernatant. Purification was achieved by stepping up the acetonitrile concentration in the 10 mM sodium phosphate buffer. BSM-C was released from the column at 40-45% acetonitrile.

## C-18 Reverse Phase Chromatography -0.1% TFA

The BSM-C containing fractions from the above column were made 15% in acetonitrile by diluting with water, then made 0.1% in trifluoroacetic acid (TFA). The C-18 column was stripped of protein by a 60% acetonitrile, 0.1% TFA wash, then equilibrated in 0.1% TFA.

Material was loaded on the column until BSM-C activity was detected in the flow through at which point 3.5 liters of 20% acetonitrile, 0.1% TFA was applied to the column followed by 5 liters of 24% acetonitrile, and 6 liters each of 30% acetonitrile, 40% acetonitrile -all 0.1% TFA. The column was then stripped of protein with 60% acetonitrile, 0.1% TFA. BSM-C activity was found in the 20% and 24% acetonitrile fractions.

The remainder of the C-18/phosphate material was loaded and the column washed and eluted as above. At this point BSM-C activity was found only in the 24% acetonitrile fraction.

## C-18 Reverse Phase Chromatography -0.5% HPBA

The above column was stripped of protein by a 60% acetonitrile, 0.1% TFA wash then equilibrated in 0.5% heptafluorobutyric acid (HFBA), pH 2.0.

The pooled eluate from the TFA column was diluted 1:1 with water, made 0.5% in HFBA, pH 2.0, and applied to the C-18 column. The reverse phase column was then eluted with 4 liters each: 20%, 25%, 30%, 34%, 38%, 42%, 50% acetonitrile with each 0.5% HFBA, pH 2.0. BSM-C was eluted with 34% acetonitrile, 0.5% HFBA, pH 2.0.

## C-18 Reverse Phase Chromatography -pH 7.16

A 5x30 cm C-18 column was prepared using Waters PrepPAK-500 C-18. 4 liters of acetonitrile were pumped through the column followed by 4 liters of 10 mM sodium phosphate, pH 7.16. The BSM-C containing fraction from the HFBA column was diluted with water to 15% acetonitrile and adjusted to pH 7.1 with concentrated sodium hydroxide. This material was applied to the column and was eluted with an acetonitrile step gradient kept 10 mM in sodium phosphate, pH 7.16. All BSM-C activity was found in the 30% acetonitrile, 10 mM phosphate fraction.

## Sephadex Chromatography in 2% Formic Acid, 0.02% Brij 35 [a]

A 5x150 cm column was poured using Sephadex G-50-120 equilibrated in 2% formic acid, 0.02% Brij 35. The 30% acetonitrile, 10mM sodium phosphate fraction was concentrated to 470 ml by lyophilization and then to 55 ml by ultrafiltration in an Amicon concentrator fitted with a 62 mm YM2 disc membrane (2,000 MW cut-off).

This material was applied to the G-50 column and eluted with 2% formic acid containing 0.02% Brij 35 at a flow rate of 1 ml/min. 20 ml fractions - (160) were collected and assayed for BSM-C. Those exhibiting activity were pooled and concentrated to 34 ml by the Amicon system described above.

(a) a polyethylene glycol alcohol ether available from Imperial Chemical Industries, Ltd., Macclesfield, Cheshire, England.

High Performance Liquid Chromatography-I (HPLC)

a) First Injection

A Beckman model 420 HPLC system was fitted with a 3.9x40 mm guard column containing Waters uBONDAPAK C-18 and a 7.8 mm x 30 cm uBONDAPAK C-18, 10 micron preparative column. "A" buffer was 0.1% TFA in Baker HPLC grade water and "B" buffer was 0.08% TFA in Baker HPLC grade acetonitrile. The column was equilibrated with repeated gradients from 100% "A" to 100% "B". Prior to injection the column was brought to 15% "B" and held there until a stable baseline was achieved. The flow rate was maintained at 5 ml/min. Finally, outflow from the first round of HPLC injections was monitored at 280 nm.

The concentrated protein solution obtained from the G-50 sizing column was mixed with 6x10E6 counts of 125₁ labeled BSM-C. 5.2 to 5.9 ml of this solution was injected via a 2 ml. injection loop as follows: 1.8 ml of solution was injected into the loop when it was in the "load" position. The loop was then moved to the "inject" position, left in this position for 1.5 minutes, returned to the "load" position and reloaded.

When the protein was completely loaded, the column was eluted by an acetonitrile linear gradient. 1 ml fractions were collected in siliconized glass tubes.

The gradient was stabilized at 15% "B" for 4 minutes following loading, then increased to 21% "B" over 6 minutes where it was held for 28 minutes. A linear gradient to 36% "B" was run over a 40 minute period. The column was then washed via a 2 minute gradient to 60% "B", held for 2 minutes and returned to 15% "B", again over a 2 minute interval. 100 ul of each 5 ml fraction was counted to locate the radioactive tracer. Six such injections were necessary to process the G-50 material. BSM-C was found to elute at 27%-28% "B" in each run. These fractions were rotovapped to approximately 2 ml each, pooled and brought to 2.7 ml using an Amicon concentrator fitted with a YM2 ultrafiltration membrane.

b) Second Injection

Two 1.35 ml injections were made onto the preparative C-18 column equilibrated with 15% "B" described above. The effluent was monitored at 214 nm. After injection, the acetonitrile concentration was maintained at 15% for 4 minutes, increased to 21% "B" over 6 minutes, held at 21% "B" for 2 minutes, and finally increased to 32% "B" over 66 minutes. Once again the flow rate was held at 5 ml/min and 1 ml fractions collected. 100 ul of each fraction was counted and the BSM-C found in the 25-26% "B" fractions. A total of 8 fractions from the two injections were rotovapped to dryness and brought back up in 8x1 ml 1% formic acid (4 ml for each injection). The two pooled effluents were then rotovapped to dryness and brought up in 2 ml each 50 mM potassium phosphate, pH 6.0 in preparation for the FPLC step.

Fast Protein Liquid Chromatography (FPLC)

A Pharmacia MonoS HR 5/5 cation exchange column was used in conjunction with the Beckman HPLC system described above. A flow rate of 1 ml/min was maintained at all times and the effluent again monitored at 214 nm. "A" buffer was 50 mM potassium phosphate, pH 6.0 while "B" buffer was 500 mM potassium phosphate, pH 6.0 -both prepared with Baker HPLC grade water.

Two 2 ml injections of the material from the second round of HPLC injections were made. 100% "A" buffer was run until no protein was detected in the flow through (21 ml). The remaining protein was eluted via a linear gradient from 100% "A" to 100% "B" over 25 minutes. All BSM-C activity was found in the flow through.

High Performance Liquid Chromatography-II

A 3.9 mm x 30 cm. Waters C-18 uBONDAPAK analytical reverse phase column was used in conjunction with the previously described Beckman HPLC system. "A" buffer was Baker HPLC water, 10 mM sodium phosphate, pH 7.1 while "B" buffer was 60% Baker acetonitrile, 10 mM sodium phosphate, pH 7.1 The effluent was monitored at 214 nm and the flow rate held constant at 2 ml/min.

The column was equilibrated in 100% "A" and 23 ml of material from the FPLC step loaded on the C-18 column in two runs -six injections of 2.25 ml for each. The concentration of "B" was increased linearly to 40% over a 5 minute period and then to 55% over an additional 30 minutes. BSM-C activity was found to elute at 50% "B". These fractions were rotovapped to dryness and redissolved in 2.25 ml of 0.1% TFA.

High Performance Liquid Chromatography-III

2.0 ml of this material was loaded onto the Waters analytical column. "A" buffer was Baker HPLC water, 0.1% TFA and "B" buffer was Baker HPLC acetonitrile, 0.08% TFA. The column was eluted via linear gradient (2 ml/min) to 60% "B" over a 60 minute interval. The effluent was again monitored at 214 nm. Under these conditions BSM-C was found to elute at 35% "B".

## 125ₗ Labeling of BSM-C

35 Units of BSM-C were iodinated using the chloramine T method. The BSM-C was added to 2uCi 125ₗ (Na 125ₗ New England Nuclear) on ice along with 200 ul of 40 ug/ml. chloramine T. The reaction was allowed to proceed for 10 minutes at 0°C. A small amount of the reaction mixture was removed by dipping surgical string into the flask. The string was then swirled in 1 ml of 2 mg/ml. ovalbumin. 20 ul of the ovalbumin was counted. 50 ul of 100% trichloroacetic acid (TCA) was then added to the ovalbumin. This was vortexed and centrifuged for 20 minutes. 20 ul of the supernatant was then counted. It was thus determined that 31% of the protein in the reaction mixture was iodinated after one TCA cycle.

% iodinated = [1-(cpm w/TCA) (1.02)/(cpm w/o TCA) x 100

This material was then loaded onto a 0.6 cm x 35 cm G-25 column equilibrated in phosphate buffered saline (PBS)-100 mM $K_2HPO_4$, 1.5 M NaCl, pH 7.1-containing 2 mg/ml. ovalbumin. 20 drop fractions were collected and 2 ul of each fraction counted. Those fractions containing radioactivity were pooled, and 50 ul aliquots frozen for later use.

## Determination of BSM-C Concentration

Somatomedin C concentration was determined via the Somatomedin C Radioimmunoassay Kit available from Nichols Institute Diagnostics in San Juan Capistrano, California. Dilutions were made with the reagent buffer provided with the kit. Due to low protein concentrations after the first HPLC step, dilutions thereafter were made with 1% ovalbumin in PBS.

## Determination of Protein Concentration

Protein concentration for all but the latter HPLC steps (I and II) were determined by the dye binding method of Bradford (Bradford, M. M., 1976 Anal. Biochem., 72, 248-254).

Due to the low level of protein present, the protein content of the HPLC purified material described above was determined by a ninhydrin microassay. In this assay samples, as well as a standard curve (0.5 g to 10.0 g bovine serum albumin), are hydrolyzed in 6N HCl for 22 hours at 100°C. in evacuated and sealed glass ampules which have been flushed with nitrogen. After the hydrolysis, the ampules are allowed to cool, cracked, and the hydrolysates rotovapped to dryness. A stock solution made weekly is prepared by mixing 50 mg ninhydrin, 7.5 mg hydrindantin and 1.9 ml ethylene glycol monomethyl ether. This solution is stored at 4°C. in an amber bottle. A working solution, prepared immediately before the assay, is made by adding 0.8 ml of the stock solution to 1.0 ml 4M sodium acetate, pH 5.5, and 1.2 ml distilled water. 150 ul of distilled water is added to each ampule followed by 150 ul of the working solution. The ampules are vortexed vigorously and incubated at 100°C. for 5 minutes after which time 0.5 ml of a 50% ethanol solution is added to each ampule. 15 minutes later the absorbance at 570 nm is recorded for each and a standard curve plotted.

1800 Units of purified BSM-C with a specific activity of 11,600 units BSMC/mg protein were thus obtained from 200 liters of bovine blood. The material exhibited a single peak when subjected to HPLC in each of the following systems, showing it to be homogeneously pure and free of other proteins of bovine origin:

```
System I:   C-18/silica

            "A" Buffer:  Baker Water
                         10 mM sodium phosphate
                         pH 7.1

            "B" Buffer:  60% acetonitrile
                         10 mM sodium phosphate
                         pH 7.1

            AUF        : 0.100 at 214 nm
            Flow Rate  : 2 ml/minute
            Gradient   : 0% B to 100% B over 30
                         minutes
```

```
System II: C-18/silica
           "A" Buffer:  Baker water, 0.1% TFA
           "B" Buffer:  Baker acetonitrile,
                        0.08% TFA
           AUF        :  0.100 at 214 nm
           Flow Rate  :  2 ml/minute
           Gradient   :  0% B to 60% B over
                         30 minutes
```

The purified BSM-C can be formulated according to known methods to prepare compositions of value for veterinary use. The purified BSM-C is admixed with a suitable carrier or carriers to provide compositions having a growth promoting amount of BSM-C for veterinary use. Suitable carriers are well known in the art and include both solid and liquid substances to afford compositions for parenteral administration. Solid formulations, of course, are for extemporaneous preparation of solutions prior to administration.

Suitable carriers are water, isotonic saline, isotonic dextrose, Ringer's solution, fatty oils of vegetable origin such as corn, sesame or peanut oil, and other non-aqueous carriers which will not interfere with the efficacy of the preparation and are non-toxic in the amount used (glycerol, sorbitol, propylene glycol).

For parenteral use dosages of from about $2.5 \times 10^{-5}$g (2500 mg) to about $1.4 \times 10^{-4}$ g (140,000 mg) per kg. of body weight are effective in effecting positive growth promotion.

### Sources of apparatus:

Amicon DC-30
HP50-43 hollow fiber filter
H10P3-20 hollow fiber filter
Amicon concentrator with YM2
    disc membrane

available from:
Amicon Corporation
182 Conant Street
Danvers, MA 01923

Waters Prep PAK-500 C-18
Waters uBONDAPAK C-18

HPLC columns available from:
    Waters Associates
    Milford, MA 01757

Sephadex G-50
Pharmacia MonoS HR5/5

available from:
Pharmacia Inc.
800 Centennial Ave.
Piscataway, NJ 08854

## Claims

1. Bovine somatomedin C characterized by exhibiting a single peak in each of HPLC systems I and II; said system I comprising:

C-18/silica, "A" buffer of water, 10 mM sodium phosphate, pH 7.1,

"B" buffer of 60% acetonitrile, sodium phosphate, pH 7.1,

and said system II comprising:

C-18/silica, "A" buffer of water, 0.1% TFA, "B" buffer of acetonitrile, 0.08% TFA.

2. Homogeneously pure bovine somatomedin C.

3. Bovine somatomedin C essentially free of other proteins of bovine origin.

4. Bovine somatomedin C in essentially pure form.

5. A composition providing a growth promoting amount of bovine somatomedin C according to claim 1 in admixture with a pharmaceutically acceptable carrier.